# EUROPEAN PATENT APPLICATION

(11) **EP 1 982 689 A1**
(43) Date of publication of application: **22.10.2008**
(21) Application number: 07106384.6
(22) Date of filing: 18.04.2007
(51) Int. Cl.: A61K 8/02, A61Q 19/10

(54) **Antimicrobial preservative free wipe**

(71) Applicant: The Procter and Gamble Company, Cincinnati, Ohio 45202 (US)
(72) Inventor: Casas-Sanchez, Jorge, 08820 El Prat de Llobregat (Barcelona) (ES); Herrlein, Matthias Kurt, 65719 Hofheim (DE); Rice, John Ernest Jr., West Chester OH 45069 (US); de Sa, Tanya Rachel, Bagshot Surrey GU19 5QP (GB)
(74) Representative: Borbach, Markus

(57) **Abstract**

A mild and gentle antimicrobially active wet wipe comprising a substrate and a lotion with a pH that ranges from 2.5 to 5.4 and contains no chemical preservatives.

## Description

### FIELD OF THE INVENTION

The present invention relates to a mild and gentle wet wipe with antimicrobial activity wherein the lotion of the wipe is free from all preservative chemicals.

### BACKGROUND OF THE INVENTION

Wet wipes are constructed from porous or absorbent sheets impregnated with a lotion and they are sold and stored in an air-tight container or wrapper to prevent the sheets drying out.

Wet wipes are made for a variety of uses. The two main categories of use are firstly, those for general household cleaning tasks, such as the cleaning of hard surfaces like floors or kitchen surfaces and secondly those made for personal cleansing, such as the removal of make up, or the cleaning of infants prior to the fitting of a new diaper or the simple refreshment of the skin after meals or while traveling. Wipes have also found use with feminine health and adult incontinence products.

A major proportion of the wipes intended for the cleansing of human skin are wet wipes which are designed for the use with infants and young children. They are particularly used by parents during the changing of babies to clear away any excess fecal or urine residues in the peri-anal region before applying a fresh diaper or nappy. Wet wipes are required to be effective cleaning agents while at the same time being very gentle and mild on the skin of the baby. This is especially important given that the skin of the baby around the genitals and anus can become very sensitive after extended contact with urine and fecal matter.

The dual aim of providing effective cleaning while at the same time being mild on the skin is usually a balancing act for the manufactures of wet wipes. This is because the chemical compounds required for effective cleaning and preservation of the wet wipe are often those that are the least mild on human skin.

For regulatory approval for wet wipe products there are strict limits to the growth of microbes within the lotion/substrate media that are allowed. To reach these standards all wipe products to date have required some degree of preservation, from known preservative compounds.

Several attempts have been made to reduce the impact of the presence of preservatives within wet wipes on the skin. For example, US 4,615,937 and US 4,781,974 disclose an anti-microbially active non-woven web for use in a wet wipe. The applicants achieved this by ensuring that the harsh preservative compounds were physically attached to the substrate to prevent them from remaining on the skin of the user. This was attained through chemical bonding, meaning that the preservative would remain attached to the substrate and not leach out during use. A milder preservative could then be used in the lotion itself if required.

Preparing further antimicrobial fibres/polymers and surfaces has been described extensively in the art, for example US 6,316,015 and US 6,077,319.

While this method proved effective in reducing the levels of preservatives needed in the wipes lotion it's use was limited by the expense of preparing the preservative bound substrate.

Lowering the pH of the lotion used proved to be an effective way of reducing the amount of preservative and strength of preservative. US 4,732,979 and US 4,772,501 disclose a low pH wet wipe utilizing only sorbic acid as a recognized preservative compound to achieve antimicrobial efficiency.

It would be desirable to develop a mild wet wipe that has antimicrobial activity and providing an alternative to known preservative compounds.

### SUMMARY OF THE INVENTION

The present invention is directed to an anti-microbial wet wipe comprising a substrate and a lotion whose pH is between 2.5 and 5.4 and is characterized in that the wet wipe exhibits antimicrobial activity while containing no conventional chemical preservative compounds.

### DETAILED DESCRIPTION OF THE INVENTION

All percentages are by weight of total composition unless specifically stated otherwise.

All ratios are weight ratios unless specifically stated otherwise.

The term "antimicrobial" or "antimicrobially active" when applied to a wipe in the present invention is defined as the ability successfully reduce the growth of selected bacteria and fungi for a minimum period of two weeks as set out in the antimicrobial efficacy test below.

A wet wipe is defined in general terms as a substrate impregnated with a lotion. While not limited to a particular use, the wet wipes of the present invention may be intended for the cleaning of the body. In particular the wipes may be used for the cleaning of the peri-anal area post defecation and the external genital area post urination in infants and young children. Wet wipes are typically of sufficient dimension to allow for convenient handling while being able to be easily disposed of by the sewage system (so called "flushable" wipes) or discretely disposed of in solid waste bins.

A substrate can be made from natural or man-made fibres or mixtures thereof. The substrate can be woven or, more preferably, a non woven structure. Typically the fibres for the substrate are selected from the group consisting of polyolefins, polyesters, cellulose, cellulose derivatives, viscose, rayon, polyamides, polyesteramides, polyvinyl alcohols, cotton, and combinations thereof. In an example embodiment the substrate would be constructed from a polypropylene/viscose mixture from 20-80 % polypropylene, in another embodiment, from a mixture of polypropylene and viscose with a 40-60 % ratio of polypropylene.

A non-woven substrate suitable for the present invention may be manufactured by any process well known in the art. This includes air and wet laying the webs and may utilize any bonding techniques known in the art, non limiting examples of which include chemical and thermal bonding, needle punch and hydro-entanglement. A particularly desired non woven substrate for the present invention would be one that is air laid and subsequently hydro-entangled.

In an embodiment the substrate may have a dry basis weight of between 20 grams per square meter (g/m²) and 100 g/m², in another between 30 g/m² and 75 g/m² and in a further embodiment between 45 g/m² and 65 g/m². There are various examples of suitable substrates in the patent literature, for example US 6,960,349.

A lotion as defined in the present invention is a chemical composition comprising water or another liquid solvent. Generally the lotion is of sufficiently low viscosity to impregnate the entire structure of the wipe. In another embodiment the lotion may be primarily present at the wipe surface and to a lesser extent in the inner structure of the wipe. In a further embodiment the lotion is releasably carried by the substrate, that is the lotion in carried on or in the substrate and is readily releasable from the substrate by applying some force to the substrate, for example by wiping a surface with the wet wipe.

The lotions used in the present invention are primarily although not limited to, oil in water emulsions. The amount of water in the lotion of the invention is normally, but not limited to, at least about 80 percent by weight water and maybe at least about 85, 90 or 95 percent by weight water.

The amount of lotion used on a substrate for a wet wipe can vary. Usually the amount is between 10% and 1000 % of the basis weight of the substrate, alternatively it is between 100 and 700 % and further it can be between 200 and 400 % of the basis weight of the substrate.

The lotion composition of the wipe of the present invention has a pH range between 2.5 and 5.4, the lotion may pH also range between 3.0 and 4.5, alternatively the pH may also range between 3.5 and 4.0. Low pH is known to aid the antimicrobial properties of wipes. This pH range is also known to be kinder to the skin as normal skin has a slightly acidic pH. Bodily wastes such as urine and faeces are known to raise the pH of the skin above its natural level and so an acidic wipe can counteract this effect.

This acidic adjustment to the lotion pH can be achieved with a variety of different chemical acid components. Non limiting examples of acids that can be used in the present invention are adipic acid, tartaric acid, citric acid, maleic acid, malic acid, succinic acid, glycolic acid, glutaric acid, malonic acid, salicylic acid, gluconic acid, polymeric acids, phosphoric acid, carbonic acid, fumaric acid and phthalic acid and mixtures thereof.

Suitable polymeric acids can include homopolymers, copolymers and terpolymers, and may contain at least 30 mole % carboxylic acid groups. Specific examples of suitable polymeric acids useful herein include straight-chain poly(acrylic) acid and its copolymers, both ionic and nonionic, (e.g., maleic-acrylic, sulfonic-acrylic, and styrene-acrylic copolymers), those cross-linked polyacrylic acids having a molecular weight of less than about 250,000, preferably less than about 100,000 poly (α-hydroxy) acids, poly (methacrylic) acid, and naturally occurring polymeric acids such as carageenic acid, carboxy methyl cellulose, and alginic acid

In one embodiment the acid for the present invention would be citric acid and/or citric acid derivatives.

The amount of the acid required in the lotion would be sufficient to lower pH into the desired range. This will depend on the other components in the lotion. Citric acid and/or citric acid derivatives would preferably be present in the range of between 0.01 and 2.5 %, or alternatively 0.1 and 1.5 %, or alternatively 0.4 and 0.9 % of the lotion.

The lotion may also contain salts of the acid or acids used to lower the pH, or another weak base to impart buffering properties to the wipe. The buffering response is due to the equilibrium which is set up between the free acid and its salt. This allows the wipe to maintain its overall pH despite encountering a relatively high amount of bodily waste as would be found post urination or defecation in a baby or adult. In one embodiment the acid salt would be sodium citrate. The amount of sodium citrate present in the lotion would be between 0.01 and 2.0%, alternatively 0.1 and 1.25 %, or alternatively 0.2 and 0.7 % of the lotion.

The lotion does not contain any preservative compounds. For the present invention a preservative compound is a compound that forms part of the following list:
Benzoic acid, its salts and esters, Propionic acid and its salts, Sorbic acid (hexa-2,4-dienoic acid) and its salts,4-Hydroxybenzoic acid its salts and esters, 3-Acetyl-6-methylpyran-2,4(3H)-dione (Dehydroacetic acid) and its salts, Hexetidine, Poly (1-hexamethylenebiguanide) hydrochloride, 2-Phenoxyethanol, Benzyl alcohol, Formaldehyde and paraformaldehyde, Formic acid and its sodium salt, Glutaraldehyde (Pentane-1,5-dial), (Sodium hydroxymethylglycinate), Bronopol, 2,4-Dichlorobenzyl alcohol, 3,3'-Bis (1-hydroxymethyl-2,5-dioxoimidazolidin-4-yl)- 1, 1'-methylenediurea ("Imidazolidinyl urea"), 1-(4-Chlorophenoxy)-1-(imidazol-1-yl)-3,3-dimethylbutan-2-one, 1,3-Bis (hydroxymethyl)-5,5-dimethylimidazolidine-2,4-dione, Hexamethylenetetramine (methenamine), 6,6-Dibromo-4,4-dichloro-2,2'-methylenediphenol (Bromochlorophen), N-(Hydroxymethyl)-N-(dihydroxymethyl-1,3-dioxo-2,5-imidazolinidyl-4)-N'-(hydroxymethyl) urea, Methenamine 3-chloroallylochloride, Biphenyl-2-ol (o-phenylphenol) and its salts, Biphenyl-2-ol (o-phenylphenol) and its salts, Chlorobutanol, 3,3'-Dibromo-4,4'-hexamethylene-dioxydibenzamidine (Dibromohexamidine) and its salts (including isethionate), Triclocarban, Triclosan, Alkyl (C-12) - C22) trimethyl ammonium, bromide and chloride, Benzalkonium chloride, bromide and saccharinate, 3-Iodo-2-propynylbutylcarbamate, Mixture of 5-Chloro-2-methyl-isothiazol-3(2H)-one and 2-methylisothiazol-3(2H)-one with magnesium chloride and magnesium nitrate, Inorganic sulphites and hydrogensulphites, Phenylmercuric salts (including borate), 1-Hydroxy-4-methyl-6 (2,4,4-trimethylpentyl)2-pyridon and its monoethanolamine salt, 4-Isopropyl-m-cresol, 2-Benzyl-4-chlorophenol (Chlorophene), 2-Chloroacetamide, Chlorhexidine and its digluconate, diacetate and dihydrochloride, 4,4-Dimethyl- 1,3-oxazolidine, 1,6-Di(4-amidinophenoxy)-n-hexane (Hexamidine) and its salts (including isethionate and p-hydroxy-benzoate), 3-(p-Chlorophenoxy)-propane-1,2-diol (chlorphenesin), 4-Chloro-3,5-xylenol, 3-Iodo-2-propynylbutylcarbamate, Salicylic acid and its salts, Pyrithione zinc, Sodium iodate, Thiomersal, 5-Bromo-5-nitro-1,3 dioxane, 4-Chloro-m-cresol, 1,2-Dibromo-2,4-dicyanobutane (methyldibromo glutaronitrile), 1-Phenoxypropan-2-ol, 5-Ethyl-3,7-dioxa-1-azabicyclo [3.3.0] octane, Silver chloride deposited on Titanium dioxide, Benzethonium chloride and Benzylhemiformal.

Typical preservative compounds are also listed in Annex VI of the European cosmetic directive.

The lotion may comprise many other ingredients. Non limiting examples of these include: skin conditioning agents (emollients, humectants) including, waxes such as petrolatum, cholesterol and cholesterol derivatives, di and tri-glycerides including sunflower oil and sesame oil, silicone oils such as dimethicone copolyol, caprylyl glycol and acetoglycerides such as lanolin and its derivatives, emulsifiers; stabilizers; surfactants including anionic, amphoteric, cationic and non ionic surfactants, colourants, chelating agents including EDTA, sun screen agents, solubilizing agents, perfumes, opacifying agents, vitamins, viscosity modifiers; such as xanthan gum, astringents and external analgesics.

The present inventors have prepared low pH wet wipe compositions, possessing no chemical preservative, that pass all the elements of the antimicrobial efficacy test as described below.

The following wet wipe is a non limiting embodiment of the present invention, a preservative free wet wipe with strong antimicrobial activity.
1 - A substrate constructed from a 60:40 mixture of polypropylene and viscose fibres, air laid and hydro-entangled with a basis weight of 55g/m², and
2 - A lotion with a pH between 3.5 and 4.0, comprising 325% by weight of substrate with the following ingredients:

**Table 1**

| Ingredient | Trade Name | % w/w |
|---|---|---|
| Aqua | Water | 96.3 |
| Disodium EDTA | EDTA | 0.15 |
| Xanthan Gum | | 0.25 |
| PEG-40 Hydrogenated Castor Oil | Cremophor CO40 | 0.5 |
| Bis-PEG/PPG - 16/16 PEG/PPG, | | |
| Dimethicone Caprylic capric | Abil Care 85 | 0.3 |
| triglyceride | | |
| Caprylyl glycol, Glycerin, Glyceryl | Dermasoft LP | 1.00 |
| Caprylate, Phenyl propanol | | |
| Citric Acid | Citric Acid | 0.9 |
| Sodium Citrate | Sodium Citrate | 0.6 |
| | Total | 100.00 |

The wipes embodying the present invention were compared in an antimicrobial efficacy test with three other commercially available wipes to demonstrate their properties. The overall test is made up of two sub-tests. Each of the sub-tests involves a different mixed pool culture of either bacteria or yeasts and moulds. The first is a mixture of bacteria and the second group is made up of yeasts and moulds. To successfully pass the antimicrobial efficacy test, the wipes must be effective at against both the groups of microbes. The full test is detailed below.

Soft and Gentle wipes from Seventh Generation® and are low pH babycare wipes. These wet wipes have a pH of 3.3 and contain potassium sorbate, a known chemical preservative. In the antimicrobial efficacy test (Table 3 below) they proved effective against both the bacteria groups but are ineffective against the yeast and moulds group *(Candida albicans* and *Aspergillus niger)* even with a known chemical preservative present.

Babycare Newborn wipes, are made by Mega disposables S.A. and were marketed as preservative free wipes. The results of the tests for these wipes can be seen in Table 4 below. These wipes failed the antimicrobial tests for the gram negative bacterial group and the yeasts and moulds group and Mega disposables S.A. later replaced them on the market by a preserved wipe product containing phenoxyethanol. These chemically preserved wipes are effective against all three groups and passed the antimicrobial efficacy test, see the results in Table 5 below.

The chemical preservative free wipe embodying the present invention (detailed in Table 1 above) passes the antimicrobial efficacy test. The results are shown in Table 2 below. The preservative free wipe of the present invention is strongly active against both the bacteria group and the yeasts and moulds group. These wipes provide strong antimicrobial protection without the need for additional chemical preservatives.

### Antimicrobial efficacy testing

The test carried out was equivalent to ASTM E640 - 78 "Standard Test Method for Preservatives in Water-Containing Cosmetics" (See also Cosmetic Microbiology, a practical approach. 2nd Edition. Edited by Philip A. Geis and published by CRC Press in 2006 - Chapter 5, Antimicrobial preservative efficacy) with the following modifications: Two mixed culture microbial groups were used in the test. These were chosen for their likely relevance to wet wipes. The use of mixed cultures was chosen to more accurately simulate the real world environment. Equal quantities of each culture were used in each group. The two groups consist of Bacteria: *Burkholderia cepacia, Klebsiella oxytoca, Pseudomonas flourecens, Serratia liqueficans* and *Enterobacter cloacae,* and Yeasts and Moulds: *Aspergillus niger* and *Candida albicans.*

The microbial groups were also harvested in sterile saline rather than sterile water and the test duration was 14 days (1 day = 24 hours) with results taken at 7 days and 14 days into the test. The growing media used was Modified Letheen Agar (with 1.5 % Tween (MLAT) Made from Merck 5349-24E or equivalent., 52.1 g Letheen agar Modified and 15 g Polysorbate 80 (Tween 80) were added to 1 L of de-ionised water prior to sterilisation - then pH adjusted to 7.2 ± 0.2) and Letheen Broth (with 1.5 % Tween (MLBT) prepared from Merck 53493 4G or equivalent : 37.8 g Letheen Broth Base Modified and 15 g Polysorbate 80 (Tween 80) is added to 1 L of dionised water. pH adjusted to 7.2 ± 0.2).
To pass the antimicrobial efficacy test the organism levels must show a 99.9% reduction (three log reductions) in microbes after seven days and maintain this reduction after 14 days. The organism levels must show a 90% (1 log reduction) after 7 days and maintain this reduction after 14 days for the yeasts and moulds.

### Test Results

### Wipes according the present invention

**Table 2**

| Log reduction of microbes | | |
|---|---|---|
| Sample group | 7 Days | 14 Days |
| Bacteria | 5.0 | 5.0 |
| Yeasts and Moulds | 3.4 | 3.9 |

Ingredients: see Table 1

### Soft and Gentle Baby Wipes from Seventh Generation®

**Table 3**

| Log reduction of microbes | | |
|---|---|---|
| Sample group | 7 Days | 14 Days |
| Bacteria | 5.2 | 5.2 |
| Yeasts and Moulds | 2.0 | 0.9 |

Ingredients: Water, Aloe barbadensis gel, Tocopherol acetate, Glycerin, Citric acid and Potassium sorbate.

### Baby Care Newborn wipes from Mega Disposables S.A. (preservative free)

**Table 4**

| Log reduction of microbes | | |
|---|---|---|
| Sample group | 7 Days | 14 Days |
| Bacteria | 0.0 | 0.0 |
| Yeasts and Moulds | 0.0 | 3.3 |

Ingredients: Water, Cocamidopropyl Betainamide MEA Chloride, Quaternium-80, Panthenol, Bisabolol, Ethylhexylglycerin, Butylene Glycol, Tilia Cordata, Glycerin, Salvia Officinalis, Arnica Montana, Parfum.

### Baby Care Newborn wipes from Mega Disposables S.A. (containing phenoxyethanol)

**Table 5**

| Log reduction of microbes | | |
|---|---|---|
| Sample group | 7 Days | 14 Days |
| Bacteria | 5.1 | 5.1 |
| Yeasts and Moulds | 3.9 | 3.9 |

Ingredients: Water, Cocamidopropyl Betainamide MEA Chloride, Quaternium-80, Panthenol, Bisabolol, Ethylhexylglycerin, Butylene Glycol, Tilia Cordata, Glycerin, Salvia Officinalis, Arnica Montana, Parfum.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A mild and gentle antimicrobially active wet wipe comprising a substrate and a lotion wherein the pH ranges from 2.5 to 5.4, **characterised in that** said lotion contains none of the following preservative chemical entities;
Benzoic acid, its salts and esters, Propionic acid and its salts, Sorbic acid (hexa-2,4-dienoic acid) and its salts,4-Hydroxybenzoic acid its salts and esters, 3-Acetyl-6-methylpyran-2,4(3H)-dione (Dehydroacetic acid) and its salts, Hexetidine, Poly (1-hexamethylenebiguanide) hydrochloride, 2-Phenoxyethanol, Benzyl alcohol, Formaldehyde and paraformaldehyde, Formic acid and its sodium salt, Glutaraldehyde (Pentane-1,5-dial), (Sodium hydroxymethylglycinate), Bronopol, 2,4-Dichlorobenzyl alcohol, 3,3'-Bis (1-hydroxymethyl-2,5-dioxoimidazolidin-4-yl)- 1, 1'-methylenediurea ("Imidazolidinyl urea"), 1-(4-Chlorophenoxy)-1-(imidazol-1-yl)-3,3-dimethylbutan-2-one, 1,3-Bis (hydroxymethyl)-5,5-dimethylimidazolidine-2,4-dione, Hexamethylenetetramine (methenamine), 6,6-Dibromo-4,4-dichloro-2,2'-methylenediphenol (Bromochlorophen), N-(Hydroxymethyl)-N-(dihydroxymethyl-1,3-dioxo-2,5-imidazolinidyl-4)-N'-(hydroxymethyl) urea, Methenamine 3-chloroallylochloride, Biphenyl-2-ol (o-phenylphenol) and its salts, Biphenyl-2-ol (o-phenylphenol) and its salts, Chlorobutanol, 3,3'-Dibromo-4,4'-hexamethylene-dioxydibenzamidine (Dibromohexamidine) and its salts (including isethionate), Triclocarban, Triclosan, Alkyl (C-12) - C22) trimethyl ammonium, bromide and chloride, Benzalkonium chloride, bromide and saccharinate, 3-Iodo-2-propynylbutylcarbamate, Mixture of 5-Chloro-2-methyl-isothiazol-3(2H)-one and 2-methylisothiazol-3(2H)-one with magnesium chloride and magnesium nitrate, Inorganic sulphites and hydrogensulphites, Phenylmercuric salts (including borate), 1-Hydroxy-4-methyl-6 (2,4,4-trimethylpentyl)2-pyridon and its monoethanolamine salt, 4-Isopropyl-m-cresol, 2-Benzyl-4-chlorophenol (Chlorophene), 2-Chloroacetamide, Chlorhexidine and its digluconate, diacetate and dihydrochloride, 4,4-Dimethyl- 1,3-oxazolidine, 1,6-Di(4-amidinophenoxy)-n-hexane (Hexamidine) and its salts (including isethionate and p-hydroxy-benzoate), 3-(p-Chlorophenoxy)-propane-1,2-diol (chlorphenesin), 4-Chloro-3,5-xylenol, 3-Iodo-2-propynylbutylcarbamate, Salicylic acid and its salts, Pyrithione zinc, Sodium iodate, Thiomersal, 5-Bromo-5-nitro-1,3 dioxane, 4-Chloro-m-cresol, 1,2-Dibromo-2,4-dicyanobutane (methyldibromo glutaronitrile), 1-Phenoxypropan-2-ol, 5-Ethyl-3,7-dioxa-1-azabicyclo [3.3.0] octane, Silver chloride deposited on Titanium dioxide, Benzethonium chloride and Benzylhemiformal.

2. A wet wipe of any of the preceding claims wherein the lotion contains adipic acid, tartaric acid, citric acid, maleic acid, malic acid, succinic acid, glycolic acid, glutaric acid, malonic acid, salicylic acid, gluconic acid, polymeric acids, phosphoric acid, carbonic acid, fumaric acid or phthalic acid or their derivatives or mixtures thereof.

3. A wet wipe of any of the preceding claims wherein the lotion contains an acid salt or weak base to provide buffering capacity.

4. A wet wipe of any of the preceding claims wherein the lotion contains skin moisturizing components, including surface active silicones such as dimethicone copolyol

5. A wet wipe of claim 4 wherein the skin moisturizing component comprises caprylyl glycol.

6. A wet wipe of any of the preceding claims wherein the lotion contains a surfactant.

7. A wet wipe of any of the preceding claims wherein the substrate is a non-woven material that has been wet or air laid and bonded via thermal or chemical bonding, needlepunch or hydroentanglement or any combination thereof.

8. A wet wipe of any of the preceding claims wherein the substrate is flushable.

9. A method of making a mild and gentle wipe of any of the preceding claims where the lotion defined in Claim 1 is added to a suitable substrate.

10. A wet wipe of any of the claims 1-8 for use in a personal cleansing product.
